Europäisches Patentamt

European Patent Office

Office européen des brevets

(19)

(11) Veröffentlichungsnummer : **0 400 426 B1**

(12) # EUROPÄISCHE PATENTSCHRIFT

(45) Veröffentlichungstag der Patentschrift :
**11.08.93 Patentblatt 93/32**

(51) Int. Cl.$^5$ : **C07C 209/26, C07C 211/09**

(21) Anmeldenummer : **90109519.0**

(22) Anmeldetag : **19.05.90**

(54) Verfahren zur Herstellung von alpha-, omega-Diaminen.

(30) Priorität : **30.05.89 DE 3917444**

(43) Veröffentlichungstag der Anmeldung :
**05.12.90 Patentblatt 90/49**

(45) Bekanntmachung des Hinweises auf die
Patenterteilung :
**11.08.93 Patentblatt 93/32**

(84) Benannte Vertragsstaaten :
**AT BE CH DE ES FR GB IT LI NL SE**

(56) Entgegenhaltungen :
**EP-A- 0 005 856**
**DE-A- 2 647 317**
**FR-A- 1 470 245**
**FR-A- 2 418 219**

(73) Patentinhaber : **HOECHST
AKTIENGESELLSCHAFT
W-6230 Frankfurt am Main 80 (DE)**

(72) Erfinder : **Kampmann, Detlef, Dr. Dipl.-Chem.
Friedhofstrasse 100
W-4630 Bochum 6 (DE)**
Erfinder : **Weber, Jürgen, Dr. Dipl.-Chem.
Bunsenstrasse 17
W-4200 Oberhausen 11 (DE)**
Erfinder : **Kniep, Claus
Rosenstrasse 93
W-4200 Oberhausen 1 (DE)**

EP 0 400 426 B1

Jouve, 18, rue Saint-Denis, 75001 PARIS

## Beschreibung

Die vorliegende Erfindung betrifft ein Verfahren zur Herstellung von $\alpha,\omega$-Diaminen aus $\alpha,\omega$-Dialdehyden. $\alpha,\omega$-Diamine besitzen aufgrund ihrer aus den beiden endstandigen Aminogruppen resultierenden chemischen Eigenschaften, eine große Bedeutung. Sie werden u.a. zur Herstellung von Kunststoffen, beispielsweise Polyamiden, zur Gewinnung spezieller Alkydharze und zur Herstellung von Appreturmitteln und Klebstoffen benötigt. Durch Kondensation von Diaminen mit Dicarbonsäuren erhalt man Netzmittel und Emulgatoren, die bevorzugt in der Ölindustrie Anwendung finden. Darüber hinaus eignen sich Diamine in besonderer Weise als Härtungsmittel für Epoxidharze.

$\alpha,\omega$-Diamine lassen sich auf verschiedenen Wegen herstellen. Man kann sie beispielsweise durch Reduktion von Dinitrilen erhalten, muß allerdings in Kauf nehmen, daß die als Ausgangsstoff erforderlichen Dinitrile nicht leicht zugänglich sind, sondern nur über eine vielstufige Synthese erhältlich sind. Aus diesem Grund besitzt dieser Weg keine große wirtschaftliche Bedeutung.

Behandelt man cyclische Olefine, z.B. Cyclohexen, mit Ozon, so gelangt man zu den entsprechenden Ozoniden, die mittels reduktiver Aminierung in die entsprechenden Diamine überführt werden können. Ein derartiges Verfahren ist in der US-PS 2,657,240 beschrieben. Die erzielbare Diaminausbeute beträgt lediglich etwa 30 % bezogen auf eingesetztes Cyclohexen. Einer Übertragung dieses Verfahrens in den technischen Maßstab sind nicht nur wegen der aufwendigen Herstellung und schwierigen Handhabung von Ozon, sondern auch wegen der geringen Ausbeute Grenzen gesetzt.

Die DE-OS 28 24 423 beschreibt ein zweistufiges Verfahren zur Herstellung von Diaminen durch Umsetzung von Dialdehyden mit einem Monoamin und anschließender Behandlung dieses Reaktionsproduktes mit Ammoniak und Wasserstoff in Gegenwart eines Hydrierkatalysators. Um Ausbeuten, die eine technische Nutzung des Verfahrens erlauben, zu erreichen, ist es allerdings erforderlich, das Monoamin in hohem Überschuß bezogen auf Dialdehyd einzusetzen.

Die DE-OS 26 47 317 betrifft ein zweistufiges Verfahren zur Herstellung von $\alpha,\omega$-Diaminen durch reduktive Aminierung entsprechender Dialdehyde. In einem ersten Schritt wird der Dialdehyd bei niedriger Temperatur in Gegenwart eines aus Wasser und einem organischen Lösungsmittel bestehenden Gemisches mit Ammoniak umgesetzt, wobei aus dem Dialdehyd unter Wasserabspaltung ein Diimin gebildet wird. Dieses Diimin wird in einem zweiten Schritt durch katalytische Reduktion zu dem $\alpha,\omega$-Diamin umgesetzt. Die erreichbare $\alpha,\omega$-Diaminausbeute ist in hohem Maße von der Temperatur, die bei der Diiminherstellung angewendet wird, abhängig. Je niedriger diese Temperatur gehalten wird, desto höher fällt die Ausbeute aus. Temperaturen im Bereich von -5 bis -10°C führen zu Ausbeuten zwischen etwa 80 bis 90% bezogen auf Dialdehyd. Erhöht man die Temperatur der Di-iminbildung geringfügig, beispielsweise auf +5°C, so sinkt die $\alpha,\omega$-Diaminausbeute bereits stark ab und die Bildung von unerwünschten Nebenprodukten steigt steil an. Einer Nutzung dieses Verfahrens steht die für die Diiminbildung erforderliche niedrige Temperatur entgegen. Für ein technisches Verfahren lassen sich derartig tiefe Temperaturen nur mit einem sehr hohen apparativen Aufwand realisieren.

Daher besteht die Aufgabe, ein Verfahren bereitzustellen, das die zuvor geschilderten Nachteile vermeidet, sich einfach ausüben läßt und eine Durchführung der Reaktion auch bei höheren Temperaturen gestattet.

Diese Aufgabe wird gelöst durch ein Verfahren zur Herstellung von $\alpha,\omega$-Diaminen aus $\alpha,\omega$-Dialdehyden, wobei man einen $\alpha,\omega$-Dialdehyd in Gegenwart von Wasser mit einem primären Amin umsetzt und das Reaktionsgemisch in Gegenwart eines Hydrierkatalysators mit überschüssigem Ammoniak und Wasserstoff behandelt.

Ein wesentliches Merkmal des neuen Prozesses ist die Anwesenheit von Wasser bei der Umsetzung von Dialdehyd und primärem Amin. Überraschenderweise erreicht man durch diese Maßnahme, daß sowohl der Umsatz als auch die Selektivität der Reaktion verbessert werden. Liegt, bedingt durch die Herstellung, der $\alpha,\omega$-Dialdehyd als ein Gemisch von n- und i-Dialdehyden vor, so bewirkt der Wasserzusatz ferner, daß die Bildung des gewünschten, aus dem n-Dialdehyd resultierenden Diamins begünstigt wird, während die Umsetzung des i-Dialdehyds zu dem entsprechenden Diamin zurückgedrängt wird.

Die Umsetzung des $\alpha,\omega$-Dialdehyds in Gegenwart von Wasser mit dem primären Amin kann bei 0 bis 60°C erfolgen. In vielen Fällen wird man die Umsetzung bei 10 bis 55°C durchführen. Besonders bewährt hat es sich, den $\alpha,\omega$-Dialdehyd in Gegenwart von Wasser bei 20 bis 50, vorzugsweise 30 bis 50°C mit dem primären Amin reagieren zu lassen.

Nach dem neuen Verfahren können beliebige $\alpha,\omega$-Dialdehyde umgesetzt werden, insbesondere aliphatische geradkettige, verzweigte oder cyclische $\alpha,\omega$-Dialdehyde. Hierunter fallen vorzugsweise geradkettige und cyclische mit 2 bis 16, insbesondere 4 bis 14, bevorzugt 6 bis 12 Kohlenstoffatomen.

Beispiele für $\alpha,\omega$-Dialdehyde sind 1,4-Butandial, 1,6-Hexandial, 1,8-Octandial, 1,10-Decandial, 1,12-Dodecandial, Bisformyltricyclo[5.2.1.0$^{2,6}$]-decan, insbesondere 1,6-Hexandial, 1,8-Octandial, 1,10-Decandial, 1,12-Dodecandial, bevorzugt 1,8-Octandial, 1,10-Decandial, 1,12-Dodecandial.

Die für die Umsetzung benötigten α,ω-Dialdehyde lassen sich mittels üblicher, in der Literatur beschriebener Verfahren herstellen.

Hinsichtlich der primären Amine unterliegt man keiner Beschrankung. Gut geeignet sind primäre aliphatische Amine, die geradkettig, verzweigt oder cyclisch, insbesondere verzweigt oder geradkettig sind. Besonders bewährt haben sich primäre aliphatische Amine mit 2 bis 6, insbesondere 3 bis 5 Kohlenstoffatomen.

Beispiele für primäre Amine sind Ethylamin, n-Propylamin, Isopropylamin, n-Butylamin, i-Butylamin, n-Pentylamin, 2-Methylbutylamin, 3-Methylbutylamin, n-Hexylamin, insbesondere Ethylamin, n-Propylamin, n-Butylamin, n-Pentylamin, bevorzugt Ethylamin, n-Propylamin und n-Butylamin.

Es hat sich bewährt, den α,ω-Dialdehyd mit in Wasser gelöstem primären Amin umzusetzen. Die Konzentration des primären Amins im Wasser beträgt üblicherweise 10 bis 90, insbesondere 20 bis 80, bevorzugt 30 bis 70 Gew.-%.

In einer weiteren, vorteilhaften Ausführungsform des erfindungsgemäßen Verfahrens setzt man den α,ω-Dialdehyd im Gemisch mit Wasser ein. Diese Gemische enthalten im allgemeinen 30 bis 70 Gew.-%, insbesondere 35 bis 65 Gew.-% α,ω-Dialdehyd und 70 bis 30, insbesondere 65 bis 35 Gew.-% Wasser.

Man kann den α,ω-Dialdehyd mit in Wasser gelöstem primären Amin oder ein aus α,ω-Dialdehyd und Wasser bestehendes Gemisch mit in Wasser gelöstem primären Amin zusammenbringen. Wichtig für das Gelingen der Reaktion ist es, den α,ω-Dialdehyd stets mit einer ausreichenden Menge primärem Amin reagieren zu lassen, um das gewünschte Azomethin (Schiff'sche Base) zu bilden. Das primäre Amin soll hierbei möglichst im Überschuß bezogen auf umzusetzende Aldehydgruppen vorliegen. Üblicherweise legt man die Gesamtmenge oder eine Teilmenge des primären Amins vor und dosiert den α,ω-Dialdehyd zu. Eine gute Durchmischung des üblicherweise heterogenen Gemisches ist, beispielsweise durch intensives Rühren, sicherzustellen.

Das Verfahren läßt sich diskontinuierlich oder kontinuierlich durchführen. Arbeitet man diskontinuierlich, so legt man das primäre Amin vor und setzt unter Rühren den α,ω-Dialdehyd gleichförmig oder absatzweise zu.

Arbeitet man kontinuierlich, so ist zu gewährleisten, daß dem umzusetzenden α,ω-Dialdehyd stets genügend primäres Amin als Reaktionspartner zur Verfügung steht. Die schnelle, vollständige Bildung der Schiff'schen Base ist wichtig für das Gelingen des erfindungsgemäßen Verfahrens. Andernfalls reagiert der α,ω-Dialdehyd zu unerwünschten Nebenprodukten. Hierfür sind beispielsweise Aldolisierungsreaktionen, Kondensations- und Polymerisationsreaktionen verantwortlich, die durch das primäre Amin katalysiert werden.

Man setzt den α,ω-Dialdehyd und das primäre Amin im Molverhältnis 1 : 2 bis 1 : 3, insbesondere 1 : 2,05 bis 1 : 2,4, bevorzugt 1 : 2,1 bis 1 : 2,25, ein. Es erweist sich häufig als vorteilhaft, das primäre Amin im Überschuß anzuwenden. Ein stöchiometrischer Aminüberschuß von 5, insbesondere 8, bevorzugt 10 %, ist in den meisten Fällen ausreichend.

Durch die Umsetzung des α,ω-Dialdehyds mit dem primären Amin wird unter Wasserabspaltung das entsprechende α,ω-Di-azomethin gebildet. Das anfallende Reaktionsgemisch besteht gewöhnlich aus zwei Phasen, nämlich aus einer organischen Phase und einer wäßrigen Phase. Die organische Phase enthält das α,ω-Di-azomethin, untergeordnete Mengen gelösten Wassers und gegebenenfalls Anteile des überschüssig eingesetzten primären Amins.

Die wäßrige Phase besteht neben geringen Mengen gelöstem α,ω-Di-azomethin und gegebenenfalls überschüssig eingesetzem primären Amin zum größten Teil aus Wasser, das einerseits zusammen mit den Einsatzstoffen, andererseits durch das Freisetzen von Reaktionswasser in das Reaktionsgemisch gelangt.

Das anfallende, aus zwei Phasen bestehende Reaktionsgemisch wird in einem nachfolgenden Schritt mit überschüssigem Ammoniak und Wasserstoff in Gegenwart eines Hydrierkatalysators nach Art einer aminierenden Hydrierung in flüssiger Phase zu dem gewünschten α,ω-Diamin umgesetzt. Je Mol ursprünglich eingesetztem α,ω-Dialdehyd setzt man 5 bis 50, insbesondere 10 bis 40, bevorzugt 15 bis 30 Mol $NH_3$ und mindestens die stöchiometrisch erforderliche Menge $H_2$ ein. Überschüssig eingesetzter Ammoniak und Wasserstoff können in die Reaktion zurückgeführt werden. Eine Erhöhung der Temperatur und eine Steigerung des Druckes begünstigen die Reaktion. Die Reaktionstemperatur soll 30 bis 180, insbesondere 50 bis 150, bevorzugt 80 bis 130°C und der Reaktionsdruck 0,5 bis 30, insbesondere 1 bis 15, bevorzugt 5 bis 12 MPa betragen. Will man die Umsetzung schonend durchführen, so wird man niedrigere Temperaturen beispielsweise <100, insbesondere <90, bevorzugt <80°C anwenden. Ist ein schneller Reaktionsablauf gewünscht, wird man höhere Reaktionstemperaturen, beispielsweise >100, insbesondere >110, bevorzugt >120°C einstellen. Durch Zugabe von $H_2$ wird der gewünschte Druck eingestellt.

Hinsichtlich des Reaktionsverlaufes darf angenommen werden, daß unter Einwirkung von $NH_3$ aus dem α,ω-Di-azomethin intermediär das entsprechende α,ω-Diimin gebildet wird. Aus diesem intermediär auftretenden Diimin wird durch Reduktion mit $H_2$ das gewünschte α,ω-Diamin gebildet.

Die Reaktion läßt sich sowohl diskontinuierlich als auch kontinuierlich durchführen.

Als Hydrierkatalysatoren können gängige Kontakte verwendet werden, die beispielsweise Cu, Co und/oder

EP 0 400 426 B1

Ni als hydrieraktiven Bestandteil enthalten. Besonders geeignet sind wasserbeständige Ni-haltige Katalysatoren.

Diese Katalysatoren weisen 40 bis 70 Gew.-%, insbesondere 45 bis 65 Gew.-%, bevorzugt 55 bis 62 Gew.-% Nickel bezogen auf die gesamte Katalysatormasse, auf. Als Trägermaterial werden Bimsstein, Aluminiumoxid, Kieselerde und Tonerde und $SiO_2$ in seinen verschiedenen Erscheinungsformen verwendet. Als Aktivator dienen Erdalkali, Aluminium, Zink und/oder Chrom enthaltende Verbindungen.

Auch ein trägerfreier Nickelkatalysator, beispielsweise Raney-Nickel, kann als Kontakt verwendet werden.

Die Raumgeschwindigkeit (flüssiges Produktvolumen: Schüttvolumen des Katalysators x Stunde) hängt von den gewählten Reaktionsbedingungen ab. Sie beträgt 0,1 bis 1,0, insbesondere 0,15 bis 0,5, bevorzugt 0,2 bis 0,35 je Stunde.

Der Katalysator kann fest angeordnet sein, z.B. in Form einer Schüttung, über die das Einsatzgemisch geleitet wird, oder suspendiert als Aufschlämmung vorliegen.

Beabsichtigt man die Umsetzung an einem festangeordneten Katalysator durchzuführen, so kann es vorteilhaft sein, das Einsatzgemisch durch Zusatz eines geeigneten Lösungmittels in eine homogene Form zu überführen und anschließend dem Katalysator zuzuleiten.

Es kann mitunter vorteilhaft sein, vor Durchführen der reduktiven Aminierung aus dem Einsatzgemisch eine Teilmenge der wäßrigen Phase abzutrennen oder das Einsatzgemisch durch Zusatz eines geeigneten Lösungsmittels in eine homogene Form zu überführen.

Nach einer bevorzugten Ausführungsform suspendiert man den Hydrierkatalysator und setzt das Einsatzgemisch dem aufgeschlämmten Katalysator zu. Als Suspensionsmittel kann das primäre Amin, rohes Reaktionsprodukt aber auch bereits gebildetes $\alpha,\omega$-Diamin dienen.

Bei der nach Art einer reduktiven Aminierung ablaufenden Umsetzung ist darauf zu achten, daß die Hydrierung des $\alpha,\omega$-Di-azomethin enthaltenden Reaktionsgemisches stets in Anwesenheit von überschüssigem $NH_3$ erfolgt. Dadurch wird sichergestellt, daß aus dem $\alpha,\omega$-Di-azomethin das ursprünglich eingesetzte primäre Amin abgespalten, das $\alpha,\omega$-Diimin intermediär gebildet und zu dem entsprechenden $\alpha,\omega$-Diamin umgesetzt wird.

Nach Beendigung der Umsetzung liegt ein homogenes Produkt vor, das aus dem gewünschten $\alpha,\omega$-Diamin, Wasser, dem primären Amin, überschüssigem Ammoniak und gegebenenfalls untergeordneten Mengen Wasserstoff und Nebenprodukten besteht. Die gasförmigen Bestandteile des Reaktionsgemisches werden abgetrennt. Das $\alpha,\omega$-Diamin wird durch Destillation gereinigt.

Die folgenden Beispiele belegen die vorliegende Erfindung, ohne sie zu beschränken.

## Experimenteller Teil

### Beispiel 1

In einem 2 l Dreihalskolben, bestückt mit Rührer, Tropftrichter, Tauchrohr, Thermometer und Rückflußkühler, werden 263 g (3,6 mol) n-Butylamin und 260 g Wasser vorgelegt. Eine Emulsion, bestehend aus 213 g (1,5 mol) Octandial (n/i-Verhältnis 80 : 20) und 200 g Wasser, wird innerhalb 60 Minuten über das Tauchrohr dem n-Butylamin-Wasser-Gemisch unter Rühren zugesetzt. Durch Einblasen eines Stickstoffstroms wird die im Tropftrichter befindliche Emulsion ständig aufgewirbelt, so daß eine Entmischung nicht eintritt.

Während der Zugabe der Emulsion beträgt die Temperatur 40°C. Nach Beendigung der Zugabe läßt man noch 2 Stunden unter Rühren bei 40°C nachreagieren.

Das erhaltene Reaktionsgemisch besteht aus zwei Phasen, einer oberen organischen und einer unteren wäßrigen Schicht. Es wird in einen Rührautoklaven überführt, mit 47 g eines trägerhaltigen Nickelkatalysators ( 50 bis 54 Gew.-% Ni, Handelsprodukt der Hoechst AG: RCH Ni 52/35) und dem 20 fachen molaren Überschuß an $NH_3$ versetzt. Durch Aufpressen von Wasserstoff wird ein Druck von 10 MPa eingestellt. Unter Rühren wird bei 120°C über einen Zeitraum von 4 Stunden hydriert. Das anfallende Hydriergemisch ist homogen. Laut gaschromatografischer Analyse weist es nachstehende Zusammensetzung (ohne Berucksichtigung von Wasser und Ammoniak) auf:

| | |
|---|---|
| Vorlauf | 0,1 Gew.-% |
| n-Butylamin | 51,5 Gew.-% |
| 2,5-Dimethyl-1,6-diaminohexan | 0,1 Gew.-% |
| 2-Methyl-1,7-diaminoheptan | 9,4 Gew.-% |
| 1,8-Diaminooctan | 38,2 Gew.-% |
| Nachlauf | 0,7 Gew.-% |

Nach Destillation an einer Kolonne mit 9 theoretischen Boden erhält man das Diamin in einer Ausbeute von 81,7 % (Selektivität 95 %).

**4**

Vergleichsbeispiel 1

Es wird wie in Beispiel 1 beschrieben gearbeitet, jedoch auf einen Zusatz von Wasser verzichtet.

Das anfallende Hydriergemisch weist laut gaschromatografischer Analyse die nachstehende Zusammensetzung (ohne Berücksichtigung von Ammoniak) auf.

| | |
|---|---|
| Vorlauf | 2,5 Gew.-% |
| n-Butylamin | 45,4 Gew.-% |
| 2,5-Dimethyl-1,6-diaminohexan | 3,0 Gew.-% |
| 2-Methyl-1,7-diaminoheptan | 4,7 Gew.-% |
| 1,8-Diaminooctan | 26,1 Gew.-% |
| Nachlauf | 18,3 Gew.-% |

Nach Destillation an einer Kolonne mit 9 theoretischen Böden erhält man das Diamin in einer Ausbeute von lediglich 60,2 %.

Beispiel 2

In einem 4 l Dreihalskolben, bestückt mit Rührer, Tropftrichter, Tauchrohr, Thermometer und Rückflußkühler, werden 567 g (7,76 mol) n-Butylamin und 567 g $H_2O$ vorgelegt. Eine Emulsion, bestehend aus 750 g (7,05 mol) Bisformyltri-cyclo[$5.2.1.0^{2,6}$]-decan (Rohprodukt) und 750 g Wasser, wird innerhalb 90 Minuten über das Tauchrohr dem n-Butylamin-Wasser-Gemisch unter Rühren zugesetzt. Durch Einblasen eines Stickstoffstroms wird die im Tropftrichter befindliche Emulsion ständig aufgewirbelt, so daß eine Entmischung nicht eintritt.

Während der Zugabe der Emulsion beträgt die Temperatur 40 bis 42°C. Nach Beendigung der Zugabe läßt man noch 2 Stunden unter Rühren bei 40°C nachreagieren.

Das erhaltene Reaktionsgemisch besteht aus zwei Phasen, einer oberen organischen und einer unteren wäßrigen Schicht. Es wird in einen Rührautoklaven überführt und wie in Beispiel 1 beschrieben hydriert. Das anfallende homogene Hydriergemisch weist laut gaschromatografischer Analyse die nachstehende Zusammensetzung (ohne Berücksichtigung von Wasser und Ammoniak) auf:

| | |
|---|---|
| n-Butylamin | 43,6 Gew.-% |
| Vorlauf-Isomere Monoamine | 0,2 Gew.-% |
| TCD*-Monoamin | 5,5 Gew.-% |
| Vorlauf-Isomere Diamine | 1,6 Gew.-% |
| TCD*-Diamin | 43,4 Gew.-% |
| TCD*-Hydroxyamin | 1,2 Gew.-% |
| TCD*-Diol + Höhersieder | 4,5 Gew.-% |

$$*TCD = Tricyclo[5.2.1.0^{2,6}]-decan$$

Bezogen auf eingesetzten Dialdehyd beträgt die Bis-(aminomethyl)-tricyclo[$5.2.1.0^{2,6}$]decan-Ausbeute (TCD-Diamin-Ausbeute) ca. 95 %.

Vergleichsbeispiel 2

Es wird wie in Beispiel 2 beschrieben gearbeitet, jedoch auf einen Zusatz von Wasser verzichtet.

Das anfallende Hydriergemisch weist laut gaschromatografischer Analyse die nachstehende Zusammensetzung (ohne Berücksichtigung von Ammoniak) auf:

| | |
|---|---|
| n-Butylamin | 40,3 Gew.-% |
| Vorlauf-Isomere Monoamine | 0,2 Gew.-% |
| TCD*-Monoamin | 4,9 Gew.-% |
| Vorlauf-Isomere Diamine | 1,4 Gew.-% |
| TCD*-Diamin | 38,4 Gew.-% |
| TCD*-Hydroxyamin | 1,1 Gew.-% |
| TCD*-Diol + Höhersieder | 13,7 Gew.-% |

$$*TCD = Tricyclo[5.2.1.0^{2,6}]-decan$$

Bezogen auf eingesetzen Dialdehyd beträgt die Bis-(aminomethyl)-tricyclo[5.2.1.0$^{2,6}$]-decan-Ausbeute(TCD-Diamin-Ausbeute) lediglich ca. 84 %.

**Patentansprüche**

1. Verfahren zur Herstellung von $\alpha,\omega$-Diaminen aus $\alpha,\omega$-Dialdehyden, dadurch gekennzeichnet, daß man einen $\alpha,\omega$-Dialdehyd in Gegenwart von Wasser mit einem primären Amin umsetzt und das Reaktionsgemisch in Gegenwart eines Hydrierkatalysators mit überschüssigem Ammoniak und Wasserstoff behandelt.

2. Verfahren nach Anspruch 1, dadurch gekennzeichnet, daß man den $\alpha,\omega$-Dialdehyd in Gegenwart von Wasser bei 20 bis 50°C mit dem primären Amin umsetzt.

3. Verfahren nach einem oder mehreren der Ansprüche 1 bis 2, dadurch gekennzeichnet, daß man als $\alpha,\omega$-Dialdehyd einen aliphatischen $\alpha,\omega$-Dialdehyd mit 6 bis 12 Kohlenstoffatomen einsetzt.

4. Verfahren nach einem oder mehreren der Ansprüche 1 bis 3, dadurch gekennzeichnet, daß man als primäres Amin ein primäres aliphatisches Amin mit 2 bis 6 Kohlenstoffatomen einsetzt.

5. Verfahren nach einem oder mehreren der Ansprüche 1 bis 4, dadurch gekennzeichnet, daß man den $\alpha,\omega$-Dialdehyd mit in Wasser gelöstem primären Amin umsetzt.

6. Verfahren nach einem oder mehreren der Ansprüche 1 bis 5, dadurch gekennzeichnet, daß die Konzentration des primären Amins im Wasser 30 bis 70 Gew.-% beträgt.

7. Verfahren nach einem oder mehreren der Ansprüche 1 bis 6, dadurch gekennzeichnet, daß man den $\alpha,\omega$-Dialdehyd im Gemisch mit Wasser einsetzt.

8. Verfahren nach einem oder mehreren der Ansprüche 1 bis 7, dadurch gekennzeichnet, daß man ein Gemisch aus 35 bis 65 Gew.-% $\alpha,\omega$-Dialdehyd und 65 bis 35 Gew.-% Wasser einsetzt.

9. Verfahren nach einem oder mehreren der Ansprüche 1 bis 8, dadurch gekennzeichnet, daß man $\alpha,\omega$-Dialdehyd und primäres Amin im Molverhältnis 1 : 2 bis 1 : 3 umsetzt.

10. Verfahren nach einem oder mehreren der Ansprüche 1 bis 9, dadurch gekennzeichnet, daß man das Reaktionsgemisch mit 10 bis 40 Mol NH$_3$ und mindestens der stöchiometrischen Menge H$_2$ jeweils bezogen auf eingesetzten Dialdehyd bei 30 bis 150°C und 1 bis 15 MPa in Gegenwart eines wasserbeständigen Nickel-Katalysators behandelt.

**Claims**

1. A process for the preparation of $\alpha,\omega$-diamines from $\alpha,\omega$-dialdehydes which comprises reacting an $\alpha,\omega$-

dialdehyde with a primary amine in the presence of water and treating the reaction mixture with excess ammonia and hydrogen in the presence of a hydrogenation catalyst.

2. The process as claimed in claim 1, wherein the $\alpha,\omega$-dialdehyde is reacted with the primary amine in the presence of water at 20 to 50°C.

3. The process as claimed in either of claims 1 and or both 2, wherein the $\alpha,\omega$-dialdehyde used is an aliphatic $\alpha,\omega$-dialdehyde having 6 to 12 carbon atoms.

4. The process as claimed in one or more of claims 1 to 3, wherein the primary amine used is a primary aliphatic amine having 2 to 6 carbon atoms.

5. The process as claimed in one or more of claims 1 to 4, wherein the $\alpha,\omega$-dialdehyde is reacted with the primary amine dissolved in water.

6. The process as claimed in one or more of claims 1 to 5, wherein the concentration of the primary amine in the water is 30 to 70% by weight.

7. The process as claimed in one or more of claims 1 to 6, wherein the $\alpha,\omega$-dialdehyde is used as a mixture with water.

8. The process as claimed in one or more of claims 1 to 7, wherein a mixture of 35 to 65% by weight of $\alpha,\omega$-dialdehyde and 65 to 35% by weight of water is used.

9. The process as claimed in one or more of claims 1 to 8, wherein the $\alpha,\omega$-dialdehyde and the primary amine are reacted in the molar ratio of 1 : 2 to 1 : 3.

10. The process as claimed in one or more of claims 1 to 9, wherein the reaction mixture is treated with 10 to 40 moles of $NH_3$ and at least the stoichiometric amount of $H_2$, relative in each case to the dialdehyde used, at 30 to 150°C and 1 to 15 MPa in the presence of a water-resistant nickel catalyst.

**Revendications**

1. Procédé pour la fabrication d'$\alpha,\omega$-diamines à partir d'$\alpha,\omega$-dialdéhydes, caractérisé en ce que l'on fait réagir un $\alpha,\omega$-dialdéhyde avec une amine primaire en présence d'eau et l'on traite le mélange de réaction par un excès d'ammoniac et l'hydrogène en présence d'un catalyseur d'hydrogénation.

2. Procédé selon la revendication 1, caractérisé en ce que l'on fait réagir la formule dialdéhyde avec l'amine primaire en présence d'eau à 20-50°C.

3. Procédé selon une ou plusieurs des revendications 1 à 2, caractérisé en ce que l'on utilise comme $\alpha,\omega$-dialdéhyde un $\alpha,\omega$-dialdéhyde aliphatique en $C_6$-$C_{12}$.

4. Procédé selon une ou plusieurs des revendications 1 à 3, caractérisé en ce que l'on utilise comme amine primaire, une amine aliphatique primaire en $C_2$-$C_6$.

5. Procédé selon l'une ou plusieurs des revendications 1 à 4, caractérisé en ce que l'on fait réagir l'$\alpha,\omega$-dialdéhyde avec l'amine primaire dissoute dans l'eau.

6. Procédé selon une ou plusieurs des revendications 1 à 5, caractérisé en ce que la concentration de l'amine primaire dans l'eau est de 30 à 70% en poids.

7. Procédé selon l'une ou plusieurs des revendications 1 à 6, caractérisé en ce que l'on utilise l'$\alpha,\omega$-dialdéhyde en mélange avec l'eau.

8. Procédé selon une ou plusieurs des revendications 1 à 7, caractérisé en ce que l'on utilise un mélange de 35 à 65% en poids d'$\alpha,\omega$-dialdéhyde et 65 à 35% en poids d'eau.

9. Procédé selon une ou plusieurs des revendications 1 à 8, caractérisé en ce que l'on fait réagir l'$\alpha,\omega$-dial-

déhyde et l'amine primaire dans un rapport molaire de 1:2 à 1:3.

10. Procédé selon une ou plusieurs des revendications 1 à 9, caractérisé en ce que l'on traite le mélange de réaction avec 10 à 40 mol de $NH_3$ et au moins la quantité stoechiométrique de $H_2$, chaque fois par rapport au dialdéhyde utilisé, à 30-150°C et sous 1-15 MPa en présence d'un catalyseur au nickel résistant à l'eau.